# Europäisches Patentamt
## European Patent Office
## Office européen des brevets

(11) Publication number: **0 003 003**
**B1**

(12) # EUROPEAN PATENT SPECIFICATION

(45) Date of publication of patent specification: **14.04.82**

(51) Int. Cl.³: **A 61 L 9/04**

(21) Application number: **78810033.7**

(22) Date of filing: **22.12.78**

(54) System and method for dispensing volatile airtreating agents.

(30) Priority: **27.12.77 US 864980**

(43) Date of publication of application:
**11.07.79 Bulletin 79/14**

(45) Publication of the grant of the patent:
**14.04.82 Bulletin 82/15**

(84) Designated Contracting States:
**CH DE FR GB IT LU**

(56) References cited:
**US - A - 3 567 118**
**US - A - 3 578 545**
**US - A - 3 685 734**
**US - A - 3 688 985**
**US - A - 3 815 828**

(73) Proprietor: **Airwick AG**
**Postfach**
**CH-4002 Basel (CH)**

(72) Inventor: **Lee, Ping I., Dr.**
**38-1/2 Wolden Road**
**Ossining New York 10562 (US)**
Inventor: **Kleiner, Eduard K.**
**207 East 74th Street**
**New York N.Y. 10021 (US)**

(74) Representative: **Schirner, Rolf et al,**
**Patentabteilung der CIBA-GEIGY AG Postfach**
**CH-4002 Basel (CH)**

## System and method for dispensing volatile airtreating agents

This invention relates to a dispensing system for volatile airtreating agents comprising a closed reservoir, a hydrophilic membrane forming at least a portion of the wall surface of said reservoir and being in contact with the atmosphere. and a volatile airtreating agent in aqueous medium contained in said reservoir in contact with said hydrophilic membrane.

A dispensing system of this type is described in U.S. Patent 3,669,637 of Klass et al. In this known system containers equipped with membranes, for instance of water-soluble cellulosic polymers, polyvinyl alcohol or gelatin are filled with a liquid odorant, e.g. a mercaptan which permeates at a constant rate through the membrane into a confined stream of propane gas or a natural mixed gas. Very small amounts of mercaptan vapors which permeate through the membrane while the latter is in direct contact with the liquid compound suffice to impart the desired odor to the gas, thus permitting rapid detection of leaks in a gas pipe.

However, the membranes recommended by Klass et al do not permit a satisfactory permeation by vapors of perfume (i.e. a mixture of essential oils) when in contact with the dry liquid perfume.

Moreover, in U.S. patent 3,815,828 of Engel, it has been recommended to use a liquid-impervious membrane of a water-insoluble vinyl polymer or copolymer, water-insoluble polymethane or the like in contact with an aqueous emulsion of volatile matter, especially an odoriferous material formed with the help of a surfactant. Vapors of the odoriferous material will permeate the membrane and are gradually released into the surrounding atmosphere as a dry gas, over a period of time which is greater than the time required for an equal amount of the odoriferous material to volatilize in its free unconfined state. However, the permeation rate of the odoriferous material through the membrane is often too low and must be enhanced by impregnating the external surface of the membrane with an aqueous emulsion containing a similar or dissimilar volatile matter, and drying the membrane until the external surface is dry to the touch. Of course, this complicates the manufacture of such dispenser systems considerably.

On the other hand, liquid-wick types and the solid air-treating agents, i.e. solid compositions from which volatile air-treating materials slowly vaporize when the solid is exposed to air are predominant among the air-treating systems, and in particular room deodorizers and the like, the same and manufacture of which has expanded substantially with the result that a large variety of air-treating products or agents have been developed for commercialization. Such air-treating agents, described for instance in US Patents Nos. 2,691,615 and 2,929,055, comprise a solid gel containing volatile air-treating materials.

Air-treating systems of this type have many advantages; however, while being satisfactory in use in many respects, they generally exhibit a less satisfactory release mode. Thus, their rate of release of active ingredient is initially high, but suffers a rapid decline with the passage of time. Such a pattern of exponential decay is often referred to as "first-order decay". As a result, the amount of released agent may initially exceed the amount required for effective air treatment, while at some later time it may be inadequate for the task. Furthermore, the release rates of such systems will be affected by temperature and humidity changes, thereby introducing additional variables that must be considered by the manufacturer. In addition, fractionation of the components of a perfume consisting of essential oils is seen to occur, thereby further increasing the possibility of erratic release patterns. Thus, the pleasant fragrance that may initially be present will vary and disappear with the passage of time and with the resultant change in concentration of the various essential oil components. Correspondingly, the effective odor counteraction that may be achieved initially will also vary and diminish with time. These effects are seen to occur as the concentration of the lower boiling components diminishes in favor of that of the higher boiling components.

Various systems which are seen to exhibit such "first-order" decay are disclosed in US Patents 3,016,199; 3,400,890; 3,596,833; 3,567,118; 2,481,296; and 3,578,545. The element common to each of these systems is that the active ingredient is homogeneously dispersed or dissolved through the basic matrix. This matrix can be as thin as a membrane (3,567,118) or as thick as a chunk of gel. The kinetics of release from such a matrix depend greatly on the geometry and loading of the system. As noted above, these systems exhibit exponential decay of release rate with respect to time. Gradually diminishing and varying odorant level and odor counteraction are observed rather than the desired constant, uniform, controlled release of fragrance.

Moreover, all of the known systems giving off dry fragrance vapors suffer from the drawbacks that it is difficult to determine at which point in time the system is exhausted, and there is left a container filled with an undesirable liquid residue the disposal of which presents problems.

Also, it will easily occur that a dispenser is discarded while still retaining in its liquid filling a considerable amount of expensive essence or the like odoriferous airtreating agent.

It is, therefore, a main object of this invention to provide a system for delivery of volatile airtreating agents which provides a substantially uniform, controlled release of said agents to the atmosphere, i.e. a "Zero order" release.

It is still a further object to provide a system which substantially minimizes fractionation among any essential oils present in said volatile airtreating agents and between the water and said essential oils.

It is yet another, preferred object of the invention to provide a system of the type described which makes it easy to detect exhaustion of the volatile airtreating agent therein, thereby avoiding waste of expensive agent.

It is finally an object of the invention to provide a system of the type described which is easily disposed of, being emptied of all liquid contents.

These objects are attained, according to the invention, in a dispensing system for volatile airtreating agents comprising a closed reservoir, a hydrophilic membrane mechanically resistant to water up to 60°C having an equilibrium water content of at least about 15% by weight calculated on the total weight of the membrane forming at least a portion of the wall surface of said reservoir and being in contact with the atmosphere, and a volatile airtreating agent present in aqueous medium contained in said reservoir in contact with said hydrophilic membrane; said system providing uniform release of both said water and said volatile airtreating agent such that said reservoir is substantially devoid of both components at the conclusion of the release period, and substantially minimizing the fractionation among any essential oils present in said volatile airtreating agent and between the water and said essential oils.

Preferably, the walls of the reservoir other than the portion thereof constituted by the membrane are collapsible, and the liquid thus fills the reservoir at all times until consumption is complete. According to another embodiment, the walls of the reservoir are constituted by the membrane and are collapsible.

The operation of the membrane involves selective sorption of air-treating agent and water into the membrane at the internal surface of the latter, selective diffusion or flow through the membrane and then desorption of vapors from the external membrane surface into the air.

By using a hydrophilic membrane as part of the reservoir wall, constant release of the aqueous-based air-treating agent is attained. The presence of the water in the liquid phase is essential in that it plasticizes the membrane and lowers the glass transition temperature of the membrane material so that the diffusive transport rate of the perfume through the membrane can be of practical value in freshening the air in a closed room. The hydrophilic membrane as defined meters the transport of aqueous-based perfume from the enclosed reservoir at a constant rate for a prolonged and controllable period of time. This is in contrast to the commercially available known systems which, as previously noted, exhibit a gradually diminishing level of active ingredient. Furthermore, the kinetics of release do not depend of the geometry and loading of the system. Rather, the total period of release depends solely on the size of the reservoir.

In addition, the controlled release of aqueous-based air-treating agent by the use of a hydrophilic membrane causes only a negligible amount of fractionation of the essential oil components of the perfume present. Thus, contrary to conventional wick systems, for example, which exhibit fast release of the low boiling components followed by the components boiling at medium and high temperature ranges, the instant systems exhibit similar percentage releases for all three portions through the swollen hydrophilic membrane. As a result, there is a uniformity of fragrance throughout the entire period of activity of the dispenser. The initial, desired fragrance is substantially maintained without the variations that would result from component fractionation. Likewise, the desired and expected level of odor counteraction is maintained without the diminution thereof resulting from component fractionation.

It is also possible to reduce or eliminate completely fractionation between the water and the essential oil type agent as a result of proper formulation of the system. Thus, undesirable release modes wherein only a portion of the essential oil is released before the water content is depleted or wherein the essential oils release and deplete faster than water are avoided. Accordingly, the unnecessary waste of essential oil and possible insufficient release rate of the first of these modes as well as the initially higher than required release and subsequent diminished fragrance level of the second mode are substantially eliminated. The instant system thus establishes that the release rates of the water and essential oil are proportional to their weight fractions by a similar constant so as to avoid the undesirable release patterns described hereinabove.

In the dispenser system according to the invention, rigid containers wherein the membrane represents one side thereof as well as collapsible containers can be used. The containers are preferably fitted with means for vacuum relief.

All conventional volatile airtreating agents of the essential oil type, in particular perfumes, are applicable for use in the instant systems. A wide variety of such materials are known to those skilled in the perfuming arts. They may comprise one or more natural materials or synthetic aromatic agents or mixtures of the two.

Such volatile airtreating agents are primarily perfumes of which a representative selection is given in British patent specification 1,336,495 of CIBA-GEIGY AG, Basel, Switzerland, on page 5, lines 92 to 106 and in British patent specification 1,517,410 to S. C. Johnson & Son, Inc. Racine, Wisconsin in Table I published September 2, 1977).

Furthermore, these agents comprise synthetic "perfumes" being mixtures of a base and a head

**0 003 003**

note selected from the deodorants and reodorants listed in British patent specification 1,432,163 of CIBA-GEIGY AG beginning in line 39 of page 3 and ending in line 29 on page 6; similar compositions are also described in German patent 1,617,018 of Rudolf RANDEBROCK, Garstedt, Germany.

The volatile airtreating agent must be dispersed in an aqueous dispersion medium in the form of a solution or emulsion or simultaneously part of its components dissolved and the remaining components emulsified. The aqueous dispersion medium can be water or a water-solvent mixture of water with alcohols, glycols, ketones, aldehydes, and the like, e.g. water-ethanol, water-glycerol, water-ethylene glycol, water-cellosolve, water-acetone, water-acetaldehyde, and the like. An emulsion of hydrophobic essential oil in water or the aforesaid water-solvent mixtures can be formed utilizing an appropriate emulsion system. Various optional ingredients may also be included such as surfactants, thickeners, dyes, stabilizers, and the like. The concentration of the air-treating agent may be selected in accordance with the particular needs with regard to intensity and duration of fragrance, and the like.

Preferably, the aqueous dispersion contains, per 100 parts by weight thereof, from 0.5 to 25 parts by weight of the air-treating agent and correspondingly from about 95 to 45 parts by weight of water or a liquid water/organic solvent mixture.

Especially preferred are dispenser systems according to the invention in which the air-treating agent is an essence, in particular a floral or herbal or wood essence, and in particular those systems in which the essence is present in an amount of from 0.5 to 5 parts by weight, and correspondingly the aqueous dispersion medium is present in an amount of about 97 to 80 parts by weight, in every 100 parts by weight of the aqueous dispersion.

The hydrophilic membrane is selected so as to exhibit good mechanical strength and, most importantly, a minimum equilibrium water content of 15%, and preferably above about 30%. Such values are required in order to maintain effective essential oil permeability. Applicable hydrophilic membranes include cellophane; Cuprophan (cellulosic membrane prepared by cuprammonium process); high molecular weight, at least 98% to fully hydrolyzed polyvinyl alcohol (e.g. manufactured by Mono-Sol Division of Chris-Craft Industries, Inc.); crosslinked polyvinyl alcohol; hydrogels; hydrophilic groups-containing polyvinyl chloride with inert absorptive filler (e.g. manufactured by Amerace Corp., Butler, N.J.); cellulose triacetate; copolyether polycarbonate; polyethylene glycol-polyethylene terephthalate block copolymer, [see Lyman et al, Biochemistry 3, 985 (1964)]; hydrophilic polyurethane (see U.S. Patent 3,822,238); and the like.

Applicable hydrogels are disclosed in numerous references including US Patents 3,520,949, 3,632,416, 3,641,237, 3,721,657, 3,784,540, 3,929,741, 3,947,401, and others. Such hydrogels are polymers of monomers containing at least one hydrophilic group which polymers have been crosslinked with a polyunsaturated crosslinking agent. Such hydrophilic monomers include hydroxyalkyl acrylates and methacrylates, diacetone acrylamide acrylamide, methacrylamide, acrylamido propane sulfonic acid, 2-(alkoxy) ethyl acrylates and methacrylates, alkyl aminoalkyl methacrylates, ethylenically unsaturated carboxylic acids, vinyl pyrrolidone, diethylene glycol monoacrylate, diethylene glycol monomethacrylate, glyceryl methacrylate, pentaerythritol methacrylate, and the like. Typical crosslinking agents are vinylic, vinylidenic or allylic in nature and include polyunsaturated polyesters formed between a polyhydroxy alcohol and an ethylenically unsaturated carboxylic acid, aliphatic polyvinylic monomers, aromatic polyvinylic monomer, polyalkyl monomers, allyl-vinyl monomers, methacrylic acid anhydride, alpha, omega-diamino acrylamides, and the like. The aforementioned monomers are only exemplary of the large number and variety of hydrogel components known to those skilled in the art which can be utilized in the instant invention.

In addition, the hydrophilic membranes can be further supported by macroporous materials such as woven and non-woven fabrics, filter paper, cardboard or macroporous plastic materials such as a porous polyvinyl chloride sheet, a porous polypropylene sheet, and the like. In general, any macroporous sheet which does not affect the release kinetics of the hydrophilic membrane system can be used.

Preferred membranes have an equilibrium water content of 30% or higher. The thickness of the membrane preferably ranges from 0.005 to 0.05 cm. The membrane should preferably show from 120 to 200% and in most preferred cases from 140 to 180% swelling in contact with water at room temperature.

With regard to the rate of release, the desired constant rate can be controlled by the type of hydrophilic membrane selected and the membrane thickness and surface area. The total period of release will be determined by the amount of aqueous-based air-treating agent. Due to the difference in permeation rate of water and essential oils through any specific water-swollen hydrophilic membrane, it is necessary in the design of a particular unit to consider the amount of essential oil that would be released in vapor form as long as the membrane is in a water-swollen state. This determination would yield an optimum composition of essential oil in water and thereby eliminate undesired waste or deficiency of active ingredient.

The container adopted to contain the instant system can be, for instance, a rigid container wherein the membrane serves as one of the walls. The membrane can be supported in any convenient manner. The container should desirably be fitted with means for vacuum relief so as to avoid the formation of a partial vacuum with the passage of time, such means including pinholes, one-way

4

valves, capillary tubes or the like. The container can also be prepared in soft, collapsible form such as a plastic bellow or a plastic packet or envelope. The collapsible construction removes the necessity for including vacuum relief means.

Although the previous discussion has made primary reference to essences as the air-treating material, it should be noted that the instant invention is equally applicable to the dispensing of disinfectants, insecticides, respiratory medicines, and the like, especially in mixture with an essence.

The following examples will further illustrate the embodiment of the invention. In these examples, all parts and percentages given are by weight unless otherwise noted.

The essence used in these examples has a minty herbal fragrance. The preservative used in Examples 1 to V consisted of 65% methyl-paraben and 35% propyl-paraben.

As emulsifiers there were used
(X)     octylphenolpolyethylene oxide (Triton X 100)
(Y)     triethanolamine laurylsulfate (Maprofix TLS 500) and
(Z)     phosphatide (Alcolec 329) in the weight ratios stated in each example.

Example I
This example illustrates a typical system of the instant invention.
For purposes of this example, the following hydrophilic membranes were utilized:

|   | Membrane | Thickness (Mils) | Degree of Swelling (%) | Equilibrium Water Content (%) |
|---|---|---|---|---|
| 1 | Hydroxyethylmethacrylate hydrogel | 6.8 | 35.6 | 26.3 |
| 2 | Hydroxyethylmethacrylate hydrogel | 8.0 | 35.6 | 26.3 |
| 3 | Cellophane | 3.0 | 117.2 | 53.95 |

(1 mil $\cong$ 0.0025 cm)

in conjunction with the following aqueous-based air-treating material

|   | Parts |   |
|---|---|---|
| Water | 95.0 | |
| Essence | 1.0 | |
| Surfactant | 1.5 | (X:Y:Z = 0.7:0.6:0.2 parts) |
| Color (green dye) | 0.1 | |
| Preservative | 0.1 | |
| Cellosolve-acetaldehyde blend (1:1 parts) | 2.3 | |

The fragrance release studies were conducted in a membrane cell designed with a small capillary connected to the atmosphere in order to prevent the formation of a partial vacuum due to release of the air-treating material. Pre-swollen membranes were mounted on the cell and the liquid introduced thereafter. The cell was inverted to bring the liquid into contact with the membrane. The loaded cells were then enclosed in a chamber where air was blown through at a predetermined rate. The temperature was maintained at 26°C and the humidity at 71%. The release rate was determined gravimetrically for a period of time up to about 220 hours, i.e. prior to a complete depletion of the reservoir. As a control, an open cup of air treating material was subjected to the same atmospheric conditions.

The following results were obtained:

5

# 0 003 003

|  | Air Flow Rate (l/min.) | Release Rate ($10^{-3}$ g/cm² hr.) |
|---|---|---|
| Open Cup | $< 3 \times 10^{-3}$ | 6.154** |
| Membrane # 1 | $< 3 \times 10^{-3}$ | 4.118 |
| Membrane # 2* | $< 3 \times 10^{-3}$ | 2.927 |
| Open Cup | 10 | 12.50** |
| Membrane # 1 | 10 | 4.634 |
| Membrane # 2* | 10 | 4.146 |
| Membrane # 3 | 10 | 7.5 |

\* Test conducted in completely sealed cell.

\*\* Initial Rate.

The above noted results clearly illustrate the desired reduction in initial release rate exhibited by the systems of this invention. Furthermore, the results indicate that the fragrance release through the instant membrane devices is "zero-order" in contrast to the open cup evaporation which exhibits "zero-order" release only during the initial stage.

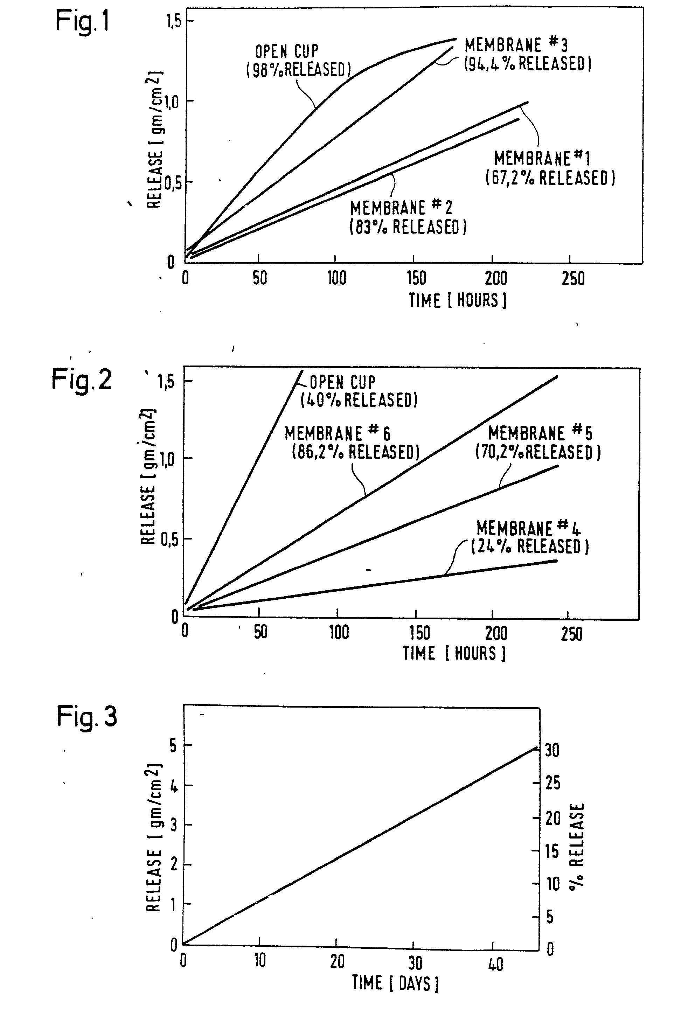

The latter result is depicted graphically in Fig. 1 which presents a plot of release (gm/cm²) versus time (hours) at an air flow rate of 10 litre/min. Thus, it is seen in Fig. 1 that the systems as reflected in membranes 1—3 provide a substantially controlled, constant rate of release as contrasted with the open cup which exhibits a significantly decreased release with the passage of time.

Example II

The procedure of Example I was repeated utilizing the identical aqueous air-treating material and conducting the test procedure at 26°C. and at an average humidity of 59%. Each membrane was tested for at least 200 hours, with several being tested up to 400 hours.

The following tables describe the tested membranes and the test results obtained therewith.

| # | Membrane | Thickness (mils) | Degree of Swelling (%) | Equilibrium $H_2O$ Content (%) |
|---|---|---|---|---|
| 4 | Crosslinked block polymer of 60% 2-hydroxy-ethylmethacrylate (A) and 40% isocyanate-terminated polyether-bisurethane (B) (HEMAC) | 10.0 | 20 | 16.67 |
| 5 | UV cured 70 (A) : 30 (B) (HEMAC) | 3.5 | 21 | 17.36 |
| 6 | 80 (A) : 20 (B) HEMAC | 10.0 | 37 | 27.00 |

6

| | Air Flow Rate (I/min.) | Release Rate ($10^{-3}$ g/cm² hr.) |
|---|---|---|
| Open Cup | > 12 | 20.7 |
| Membrane # 4 | > 12 | 1.449 |
| Membrane # 5 | > 12 | 4.000 |
| Membrane # 6 | > 12 | 6.418 |

Once again the desired reduction in initial release rate and the release through the membranes in a "zero-order" mechanism were noted. Graphical depictions of the performances of membranes 4—6 are presented in Fig. 2.

## Example III

A sealed container was prepared whereby a filter paper dip coated with hydroxyethylmethacrylate monomer was then cured with ultra-violet radiation directly onto the inside of a front perforated panel, the resulting membrane having a thickness of about 10 mils (about 0.025 cm) and an effective area of 12.932 cm². The aqueous air-treating material of Example I was then introduced into the container by syringe and the pinhole sealed. Release measurements were conducted at room temperature and a humidity of 61% and with substantially no air flow. The unit was run for a period of 44 days and was found to exhibit a constant release rate of 0.1089 g/cm²day and a total release of 1.4082 g/day. A graph of the release rate, noted as Fig. 3, revealed a "zero-order" release mechanism.

## Example IV

The testing apparatus, procedure and aqueous air-treating material of Example I were utilized herein in connection with a microporous polyvinylchloride film (#7) containing an inert, absorptive, inorganic filler, said membrane having a 20 mil (0.050 cm) thickness, a 150% degree of swelling and a 60% equilibrium water content. The release rate, as determined over a period of 65 hours at room temperature, 61% humidity and in the absence of air flow, was found to be 0.39 g/cm²day. A plot of the individual readings revealed a "zero-order" release mechanism.

## Example V

The test procedure of Example I was repeated utilizing the following aqueous air-treating materials and hydrophilic membranes.

| | Water Content (parts) | Essence Content (parts) | Other Ingredients (parts) |
|---|---|---|---|
| B | 96 | 0.85 | 3.15 surfactant, color and preservatives |
| C | 96 | 0.85 | 3.15 surfactant + cellulosic thickener |
| D | 49.5 | 25.0 | 25.5 surfactant + non-volatile thickener |

(Parts are by weight)

| # | Membrane | Thickness (mils) | Degree of Swelling (%) | Equilibrium Water Content (%) |
|---|---|---|---|---|
| 8 | Cuprophan 150 PM | 0.984 | 116.5 | 53.8 |
| 9 | Cuprophan 250 PM | 1.5 | 115.0 | 53.5 |
| 10 | Water-insoluble polyvinyl alcohol (cold $H_2O$ insoluble) | 2.6 | 140.0 | 58.3 |

Each test was conducted at two temperatures, at an average humidity of 43% and with an air flow in excess of 12 l/min. Open cup controls were also included in the test procedure.
The results of these tests are presented in the following table.

| Membrane # | Air Treating Agent | Temp. (°C.) | Release Rate (gm/cm²day) |
|---|---|---|---|
| 8 | B | 23.3 | 1.1520 |
| 8 | B | 35.0 | 3.0648 |
| 8 | C | 23.3 | 1.0047 |
| 8 | C | 35.0 | 2.3460 |
| 8 | D | 35.0 | 0.7093 |
| 9 | B | 23.3 | 1.3440 |
| 9 | B | 35.0 | 2.8464 |
| 10 | B | 23.3 | 1.260 |
| 10 | B | 35.0 | 2.3520 |
| 10 | C | 23.3 | 0.9874 |
| 10 | C | 35.0 | 2.3977 |
| 10 | D | 35.0 | 0.5578 |
| 7 | C | 35.0 | 1.9583 |
| 3 | B | 35.0 | 2.6136 |
| Open Cup | B | 23.3 | 1.8240 |
| Open Cup | B | 35.0 | 3.1872 |
| Open Cup | C | 23.3 | 1.9533 |
| Open Cup | C | 35.0 | 3.4622 |

Once again, the release data for these systems revealed a "zero-order" release rate up to about 80—90% of the total release capacity.

8

## Example VI

This example illustrates a further advantage of the instant system in terms of reduced fractionation effects.

Initially, fractionation effects between essential oil components of an essence of "natural" fragrance (wood, herbal or floral) were determined. In this instance, a preswollen membrane of water-insoluble polyvinyl alcohol obtained by 99% hydrolization of polyvinylacetate (degree of swelling 140%, equilibrium water content 58.3%, wet thickness 2.6 mils, insoluble in water below 60°C) was mounted on a closed cell and the liquid content introduced thereon. The liquid content was stirred and air at a controlled rate (>11 liter/min for membrane cell and <5 liter/min for open cup) was blown across the membrane surface to accelerate release. The time course of release was followed gravimetrically. Periodic samples were taken of the liquid remaining in the cell and analyzed by gas-liquid chromatography. Comparable analyses were conducted on liquid introduced into an open cup. The essential oil components were categorized as low (0—15 minute retention time), medium (16—34 minute retention time) and high (35—44 minutes retention time).

The polyvinyl alcohol had a viscosity of 0.0113 Pas at 60°C (concentrate in water), and the same aqueous solution had a viscosity of 0.0275 Pas at 25°C.

The following formulations were utilized:

|  | Parts | |
|---|---|---|
|  | E | F |
| Essence | 0.85 | 2.55 |
| Surfactant | 1.34 | 4.02 |
| Dye | 0.50 | 0.50 |
| Preservative | 1.36 | 4.08 |
| Glycerin | — | 1.50 |
| Demineralized water | 95.95 | 87.35 |

(in E: $X:Y:Z = 68:55:11$;

in F: $X:Y:Z = 204:165:33$)

The preservative consisted of methyl-paraben 0.906, propyl-paraben 0.302, and cellosolve 0.906 parts in Example VI—E and of methyl-paraben 0.906, propyl-paraben 0.456 and cellosolve 2.718 parts in Example VI—F.

The GLC values are presented in the following tables (GLC = gas liquid chromatography).

Upon combining the results of total release by weight loss as well as the amount released per unit area for the essential oil components, the percentage essential oil released as well as the percentage released for the essential oil components is determined and are also presented in the following tables. In each instance, the open cup area was 5.1875 cm² and the membrane area was 23.7583 cm².

9

**0 003 003**

FORMULATION E

| Time (hr) | Control (open cup) | | | | | | |
|---|---|---|---|---|---|---|---|
| | GLC Analysis | | | % Release (per cm²) | | | |
| | Low (%) | Med (%) | High (%) | Low (%) | Med (%) | High (%) | % Oil Release |
| 0 | 17.9 | 71.4 | 10.6 | 0 | 0 | 0 | 0 |
| 3.92 | 17.6 | 71.2 | 11.0 | 9.59 | 8.29 | 4.55 | 8.05 |
| 23.42 | 15.5 | 69.9 | 14.6 | 37.12 | 28.89 | — | 27.39 |
| 48.75 | 9.9 | 68.9 | 21.2 | 75.03 | 56.44 | 9.69 | 54.87 |
| 71.67 | 5.8 | 67.1 | 27.1 | 89.95 | 70.89 | 20.79 | 69.43 |
| 95.26 | 3.4 | 67.0 | 29.6 | 95.34 | 76.97 | 31.48 | 75.46 |

| Time (hr) | Membrane Cell | | | | | | |
|---|---|---|---|---|---|---|---|
| | GLC Analysis | | | % Release (per cm²) | | | |
| | Low (%) | Med (%) | High (%) | Low (%) | Med (%) | High (%) | % Oil Release |
| 0 | 17.9 | 71.4 | 10.6 | 0 | 0 | 0 | 0 |
| 4.97 | 18.1 | 71.3 | 10.5 | 2.46 | 3.66 | 4.37 | 3.52 |
| 24.7 | 18.4 | 70.8 | 10.8 | 2.90 | 6.31 | 3.70 | 5.52 |
| 49.92 | 19.5 | 69.1 | 11.4 | 6.43 | 16.88 | 7.62 | 14.11 |
| 74.42 | 19.2 | 69.5 | 11.3 | 12.81 | 20.85 | 13.27 | 18.69 |
| 97.85 | 20.2 | 71.1 | 8.7 | 13.04 | 22.49 | 36.12 | 22.15 |

FORMULATION F

| Time (hr) | Control (open cup) | | | | | | |
|---|---|---|---|---|---|---|---|
| | GLC Analysis | | | % Release** (per cm²) | | | |
| | Low (%) | Med (%) | High (%) | Low (%) | Med (%) | High (%) | % Oil Release**** |
| 0 | 19.81 | 71.15 | 9.03 | 0 | 0 | 0 | 0 |
| 2.67 | 19.43 | 71.13 | 9.44 | 6.19 | 4.35 | 0.18 | 4.32 |
| 22.7 | 15.09 | 73.43 | 11.48 | 46.01 | 26.89 | 10.05 | 29.16 |
| 47.67 | 0.20 | 85.76 | 14.23 | 99.54 | 45.24 | 28.40 | 54.57 |
| 70.92 | 0.20 | 85.00 | 14.80 | 99.60 | 52.10 | 34.39 | 59.91 |

| Time (hr) | Membrane Cell | | | | | | |
|---|---|---|---|---|---|---|---|
| | GLC Analysis | | | % Release* (per cm²) | | | |
| | Low (%) | Med (%) | High (%) | Low (%) | Med (%) | High (%) | % Oil Release*** |
| 0 | 19.81 | 71.15 | 9.03 | 0 | 0 | 0 | 0 |
| 3.85 | 20.29 | 69.91 | 9.80 | 6.16 | 9.97 | 0.33 | 8.38 |
| 24.0 | 20.95 | 70.38 | 8.67 | 13.77 | 19.34 | 21.82 | 18.47 |
| 49.09 | 22.37 | 68.61 | 9.01 | 44.81 | 52.87 | 51.30 | 51.13 |
| 73.29 | 24.71 | 65.25 | 10.04 | 80.60 | 85.73 | 82.72 | 84.44 |

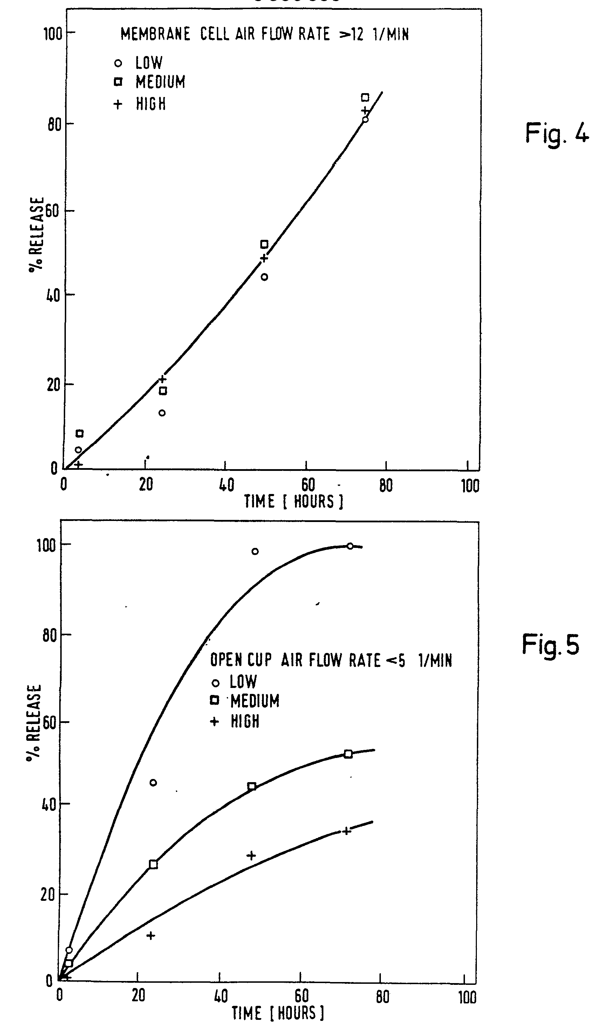

\* Data depicted in Fig. 4.

\*\* Data depicted in Fig. 5.

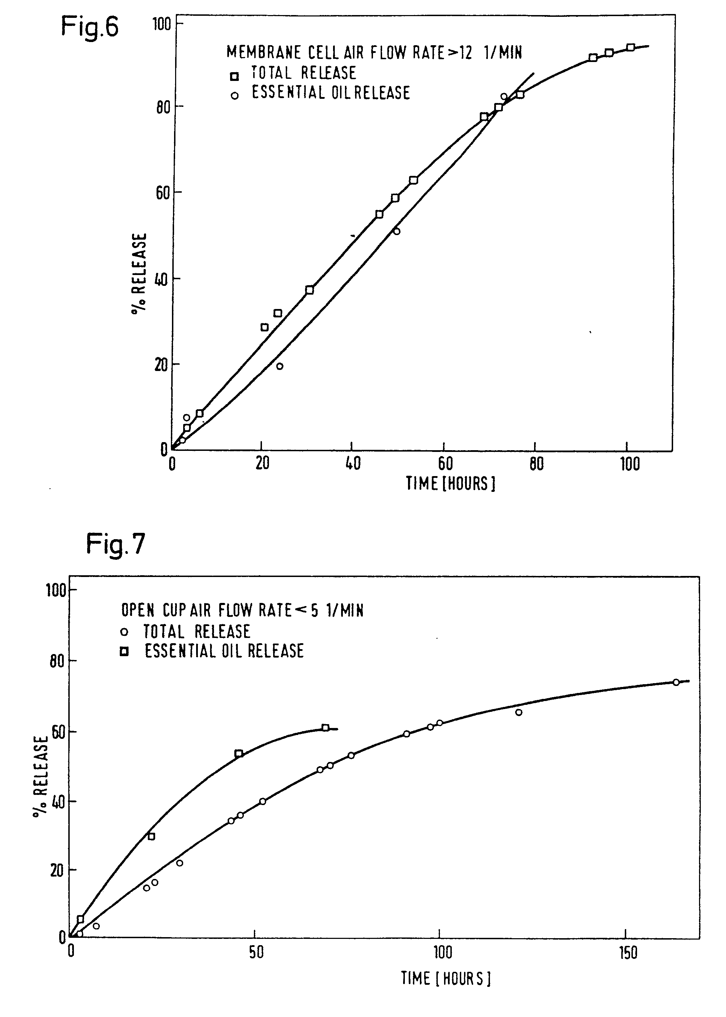

\*\*\* Data depicted in Fig. 6.

\*\*\*\* Data depicted in Fig. 7.

The data presented hereinabove thus clearly reveal the substantial reduction in essential oil and essential oil-water fractionation with the instant system. Referring specifically to the graphs (Figs. 4—7) for formulation F, linear release characteristics are exhibited. The percentage essential oil vapor release follows closely with the percentage total release (Fig. 6) indicating there is no fractionation between water and essential oil as compared to the open cup evaporation where fractionation does occur (Fig. 7). In other words, the essential oil concentration in the polyvinyl alcohol membrane system remained pretty much the same during the whole course of release study. Figures 4 and 5 further show that there is practically no fractionation among components of the essential oil in the PVA membrane system as compared to that in the open cup control where fractionation prevails.

Corresponding data were developed for formulation F utilizing the identical test procedure except for the elimination of the air blown across the membrane surface. Thus, stagnant conditions were achieved. The following results were obtained.

# 0 003 003

| Time (hr) | Membrane Cell | | | Membrane Cell | | | % Oil Release |
|---|---|---|---|---|---|---|---|
| | GLC Analysis | | | (% Release) | | | |
| | Low (%) | Med (%) | High (%) | Low (%) | Med (%) | High (%) | |
| 0 | 29.50 | 56.97 | 13.53 | 0 | 0 | 0 | 0 |
| 76.50 | 35.84 | 48.90 | 15.26 | – | – | – | 7.60 |
| 167.67 | 34.64 | 50.54 | 14.81 | 15.6 | 36.3 | 21.4 | 28.16 |
| 243.75 | 32.80 | 55.74 | 11.47 | 56.2 | 61.5 | 45.5 | 60.64 |
| 313.84 | 43.39 | 39.73 | 16.97 | 66.8 | 78.2 | 60.9 | 68.81 |

It is seen that comparable elimination of fractionation is also achieved under stagnant conditions.

Furthermore, only minimal fractionation was also observed when evergreen oil and honeysuckle oil, absent surfactant concentrations, were subjected to similar test procedures.

The membranes usable in the dispensing system according to the invention must be mechanically resistant to water up to 60°C, i.e. they should swell, but should not melt or be otherwise destroyed when in contact with the aqueous dispersion of air-treating agent below that temperature.

Summarizing, it is seen that this invention provides a unique system for the controlled release of air-treating materials. Variations may be made in proportions, procedures and materials without departing from the scope of the invention as defined by the following claims.

In particular, the system according to the invention permits easy detection of the exhaustion of the system by the fact that the reservoir is empty.

Moreover, the empty reservoir can be more easily destroyed than the known systems still containing a large proportion of the liquid filling in their interior even after exhaustion of the air-treating agent.

Preferred embodiments of the dispenser system according to the invention shall now be described in detail. These embodiments are illustrated in the accompanying drawings, in which

Fig. 8 shows in lateral view a preferred embodiment of the dispenser system;

Fig. 9 shows a bottom view of the embodiment of Fig. 8;

Fig. 10 is a sectional view of the same embodiment taken in a plane indicated by X—X in Fig. 9;

Fig. 11 is a lateral view of another embodiment of the dispenser system;

Fig. 12 is a bottom view of the embodiment shown in Fig. 11 and;

Fig. 13 is a sectional view of the embodiment of figures 11 and 12 taken in a plane indicated by XIII—XIII in Fig. 12.

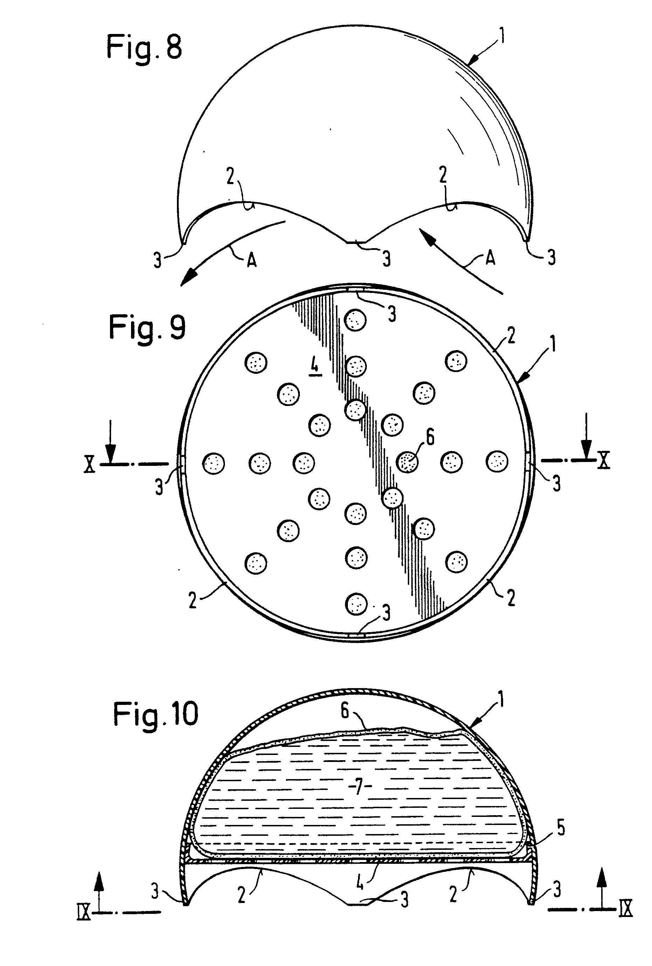

The embodiment shown in figures 8 to 10 comprises a frustospherical hull 1 the open base of which has curved cutaway wall portions 2 between which four foot portions are left on which the hull can be placed upright on a shelf or the like horizontal support. In a central plane above which the hull portion constitutes a hemisphere, a perforated plate 4 or the like grid is fastened, e.g. by thermo-welding a circumferential margin 5 thereof on the inside surface of the hemispherical portion of hull 1.

In the hemispherical space 6 enclosed between plate 4 and the dome of hull 1 thereabove, there is lodged a hydrophilic membrane 6 filled with an air-freshening liquid 7. Arrows A indicates air flowing through the windows formed by cutaway wall portions 2 of hull 1 and taking up vapors of air-freshening agent and water that have permeated the membrane 6.

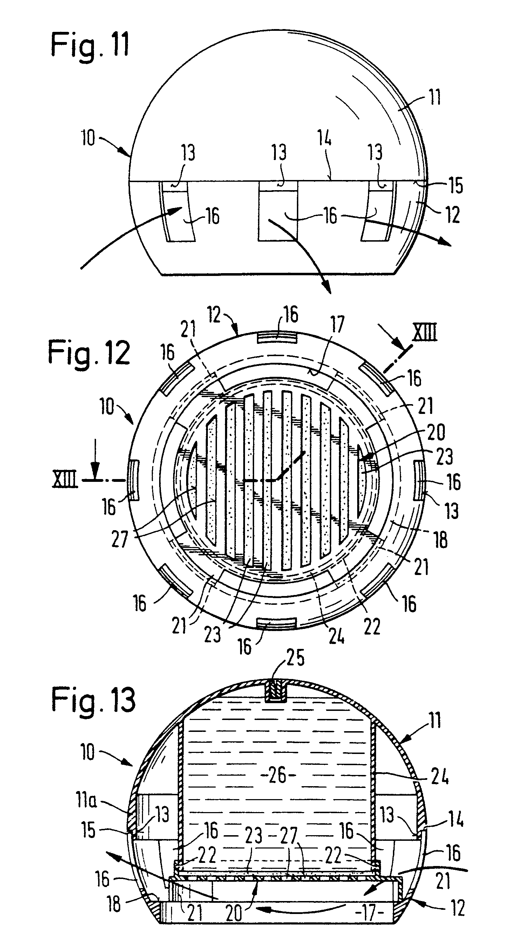

In the embodiment of Figures 11 to 13, the frustoconical hull 10 consists of an upper hemispherical half 11 as dome part and a lower frustopherical segment as base part 12. The lower annular rim portion 11a of the open end of dome part 11 bears an inner circumferential sleeve portion 13 of smaller diameter than the inner width of the upper end 14 of base part 12, whereby a shoulder 15 is formed externally about sleeve portion 13 which shoulder 15 comes to rest on the rim surface of upper end 14. Sleeve portion 13 and upper base part end 14 can be permanently fastened together by thermo-welding or gluing after the internal parts housed in the interior of hull 10 have been assembled.

The base part 12 is provided with a plurality of cut-in wall portions downward from its upper end 14 and forming windows 16 in the assembled hull 10 which permit the passage of air therethrough during use. The bottom end of base part 12 has a central opening 17 and about the latter a circumferential inner shoulder 18 is provided on the inner wall of base part 12. A slotted grid 20 or the like plate is seated by means of feet 21 on the shoulder 18 and bears an axial upwardly directed peripheral flange 22 which surrounds a hydrophilic membrane 23 placed on the grid 20 and held in

12

place thereon by a cylindrical sleeve 24 which depends from the upper inner region of the wall of dome part 11 and is preferably integral with the latter. The lower end of sleeve 24 fits snugly into peripheral flange 22 of grid 20 and urges the periphery of membrane 23 against grid 20.

At the top of dome part 11 there is provided a vacuum relief means which, in the embodiment shown, is a slit rubber plug 25 which admits air from the outside whenever a vacuum develops in the interior 26 of sleeve 24 due to diffusion of air-freshening agent and water through the membrane and evaporation of these components from the external (lower) face of membrane 23 and escape of the vapors via slots 27 of grid 20 and windows 16.

Air-freshening liquid can be filled into the sleeve interior 26 after the membrane 23 has been secured liquid-tight across the lower end of sleeve 24, with the aid of an injection means introduced through the slit of rubber plug 25.

The rubber plug 25 can also be replaced by a rubber check valve or rubber grommet or the like means of known construction.

As an alternate type of attachment of the membrane 23, the lower end of sleeve 24 can be provided with an external threading and peripheral flange 22 of grid 20 can be provided with an internal threading whereupon the membrane 23 is placed in flange 22 or on the lower open end of sleeve 24 and the latter two parts are then screw-connected together holding the periphery of membrane 23 firmly and liquid-tight between them.

For purposes of storage, the dispenser can be sealed in a vapor-tight envelope (not shown) or the like sealing-in means.

## Claims

1. A dispensing system for volatile airtreating agents comprising a closed reservoir, a hydrophilic membrane mechanically resistant to water up to 60°C having an equilibrium water content of at least about 15% by weight calculated on the total weight of the membrane forming at least a portion of the wall surface of said reservoir and being in contact with the atmosphere, and a volatile airtreating agent present in aqueous medium contained in said reservoir in contact with said hydrophilic membrane; said system providing uniform release of both said water and said volatile airtreating agent such that said reservoir is substantially devoid of both components at the conclusion of the release period, and substantially minimizing the fractionation among any essential oils present in said volatile airtreating agent and between the water and said essential oils.

2. The dispensing system of claim 1, wherein said hydrophilic membrane is selected from the group consisting of cellophane; Cuprophan; high molecular weight, fully hydrolyzed polyvinyl alcohol; crosslinked polyvinyl alcohol; hydrogels; hydrophilic groups containing polyvinyl chloride with inert, absorptive filler; cellulose triacetate; copolyether polycarbonate; polyethylene glycol-polyethylene terephthalate block copolymer; and hydrophilic polyurethane.

3. The dispensing system of claim 1, wherein said equilibrium water content is at least about 30%.

4. The dispensing system of claim 1, wherein said aqueous medium is a water-solvent mixture selected from the group consisting of water-alcohol, water-glycol, water-ketone and water-aldehyde.

5. The dispensing system of claim 2. wherein said hydrophilic membrane is cold water-insoluble polyvinyl alcohol.

6. The dispensing system of claim 1, wherein said reservoir is a rigid container having means for vacuum relief.

7. The dispensing system of claim 1, wherein said reservoir is a flexible container.

8. The dispensing system of claim 1, wherein said hydrophylic membrane is supported on a macroporous substrate.

9. The dispensing system of claim 1, wherein said air-treating material is present in an oil-water emulsion.

10. A method for the controlled, uniform, substantially constant release of volatile airtreating agents into the atmosphere comprising placing an aqueous system containing said volatile airtreating agent into a closed reservoir having as at least a portion of the wall surface thereof a hydrophilic membrane mechanically resistant to water up to 60°C with an equilibrium water content of at least about 15% by weight calculated on the total weight of the membrane, said aqueous system contacting said membrane; swelling said membrane; and allowing said airtreating agent to diffuse through said membrane and be released into the atmosphere; said aqueous phase also being transmitted through said membrane such that said reservoir is substantially empty at the conclusion of the release period.

11. The method of claim 10, wherein said hydrophilic membrane is selected from the group consisting of high-molecular weight, fully hydrolized polyvinyl alcohol; crosslinked polyvinyl alcohol; hydrogels; hydrophilic groups containing polyvinyl chloride with inert absorptive filler; cellulose triacetate; copolyether polycarbonate; polyethylene glycol-polyethylene terephthalate block copolymer; and hydrophilic polyurethane.

12. The method of claim 10, wherein said aqueous medium is a water-solvent mixture selected from the group consisting of water-alcohol, water-glycol, water-ketone and water-aldehyde.

**0 003 003**

13. The method of claim 11, wherein said hydrophilic membrane is cold water-insoluble polyvinyl alcohol.

**Patentansprüche**

1. Vorrichtung zum Abgeben flüchtiger Luftbehandlungsmittel, bestehend aus einem geschlossenen Vorratsbehälter, einer hydrophilen Membrane, die gegenüber Wasser bis zu 60°C mechanisch wiederstandsfähig ist, ein Gleichgewicht bei einem Wassergehalt von mindestens 15 Gewichtsprozent, bezogen auf das Gesamtgewicht der Membrane, aufweist, mindestens einen Teil der Wandungsoberfläche des Behälters bildet und in Kontakt mit der Atmosphäre steht, und ein flüchtiges Luftbehandlungsmittel, welches in einem mit der hydrophilen Membrane in Kontakt stehenden wässrigen Medium in dem Behälter enthalten ist, wobei die Vorrichtung eine gleichmässige Freisetzung sowohl von Wasser als auch flüchtigem Luftbehandlungsmittel bewirkt, bis der Behälter am Ende der Freisetzungsperiode weitgehend frei von beiden Komponenten ist, und die Fraktionierung von im flüchtigen Luftbehandlungsmittel vorliegenden ätherischen Oelen und zwischen Wasser und solchen ätherischen Oelen vermindert.

2. Vorrichtung gemäss Anspruch 1, worin die hydrophile Membrane aus der Gruppe, bestehend aus Cellophane, Cuprophan, hochmolekularem, vollständig hydrolysiertem Polyvinylalkohol, vernetztem Polyvinylalkohol, Hydrogelen, hydrophile Gruppen enthaltendem Polyvinylchlorid mit inertem absorptivem Füllmittel, Cellulosetriacetat, Copolyäther-Polycarbonat, Polyäthylenglykol-Polyäthylenterephthalat-Block-copolymer und hydrophilem Polyurethan, ausgewählt ist.

3. Vorrichtung gemäss Anspruch 1, worin das Gleichgewicht bei einem Wassergehalt von mindestens 30% gehalten wird.

4. Vorrichtung gemäss Anspruch 1, worin als wässriges Medium eine Mischung von Wasser mit Lösungsmittel aus der Gruppe, bestehend aus Wasser-Alkohol, Wasser-Glykol, Wasser-Keton und Wasser-Aldehyd, ausgewählt ist.

5. Vorrichtung gemäss Anspruch 2, worin die hydrophile Membrane kaltes Wasser mit unlöslichem Polyvinylalkohol ist.

6. Vorrichtung gemäss Anspruch 1, worin der Vorratsbehälter ein steifes Gefäss ist, welches Mittel für ein Vakuumrelief aufweist.

7. Vorrichtung gemäss Anspruch 1, worin der Vorratsbehälter ein flexibles Gefäss ist.

8. Vorrichtung gemäss Anspruch 1, worin die hydrophile Membrane auf ein makroporöses Substrat gestützt ist.

9. Vorrichtung gemäss Anspruch 1, worin das Luftbehandlungsmittel in einer Oel-Wasser-Emulsion vorliegt.

10. Verfahren zur kontrollierten, einheitlichen und im Wesentlichen konstanten Abgabe von flüchtigen Luftbehandlungsmitteln in die Atmosphäre durch Einbringen eines ein flüchtiges Luftbehandlungsmittel enthaltenden wässrigen Systems in einen geschlossenen Vorratsbehälter, dessen Wandungsoberfläche mindestens teilweise als hydrophile Membrane ausgebildet ist, die gegenüber Wasser bis zu 60°C mechanisch widerstandsfähig ist und ein Gleichgewicht bei einem Wassergehalt von mindestens 15 Gewichtsprozent, bezogen auf das Gesamtgewicht der Membrane, aufweist, indem das mit der Membrane in Kontakt stehende wässrige System die Membrane anschwellen und das Luftbehandlungsmittel durch die Membrane diffundieren und in die Atmosphäre entweichen lässt, wobei auch die wässrige Phase durch die Membrane gelassen wird, so dass der Vorratsbehälter am Ende der Freisetzungsperiode weitgehend leer ist.

11. Verfahren gemäss Anspruch 10, worin die hydrophile Membrane aus der Gruppe, bestehend aus hochmolekularem, völlig hydrolysiertem Polyvinylalkohol, vernetztem Polyvinylalkohol, Hydrogelen, hydrophile Gruppen enthaltenden Polyvinylchlorid mit inertem absorptivem Füllmittel, Cellulosetriacetat, Copolyäther-Polycarbonat, Polyäthylenglykol-Polyäthylenterephthalat-Blockcopolymer und hydrophilem Polyurethan, ausgewählt ist.

12. Verfahren gemäss Anspruch 10, worin als wässriges Medium eine Mischung von Wasser mit Lösungsmittel aus der Gruppe, bestehend aus Wasser-Alkohol, Wasser-Glykol, Wasser-Keton und Wasser-Aldehyd, ausgewählt ist.

13. Verfahren gemäss Anspruch 11, worin die hydrophile Membrane kaltes Wasser mit unlöslichem Polyvinylalkohol ist.

**Revendications**

1. Système de diffusion d'agents volatils de traitement de l'air comprenant un réservoir fermé, une membrane hydrophile mécaniquement résistante à l'eau jusqu'à 60°C ayant une teneur en eau à l'équilibre d'au moins 15% en poids par rapport au poids total de la membrane formant au moins une partie de la surface de paroi dudit réservoir et étant en contact avec l'atmosphère, et un agent volatil de traitement de l'air qui se trouve en milieu aqueux contenu dans ledit réservoir en contact avec ladite membrane hydrophile; ledit système fournissant une libération uniforme à la fois de ladite eau et dudit agent de traitement de l'air volatil de sorte que ledit réservoir est pratiquement vidé des deux cons-

14

tituants à la fin de la période de libération, et ledit système minimisant pratiquement le fractionnement des huiles essentielles présentes dans ledit agent volatil de traitement de l'air et entre l'eau et lesdites huiles essentielles.

2. Système de diffusion selon la revendication 1, où ladite membrane hydrophile est choisie dans le groupe comprenant la cellophane; le cuprophane; l'alcool polyvinylique complètement hydroxylé de poids moléculaire élevé; l'alcool polyvinylique réticulé; les hydrogels; le chlorure de polyvinyle contenant des groupes hydrophiles et ayant une charge absorbante inerte; le triacétate de cellulose; le copolyéther polycarbonate; le copolymère bloc de polyéthylène glycol-téréphtalate de polyéthylène; et les polyuréthanes hydrophiles.

3. Système de diffusion selon la revendication 1, où ladite teneur en eau à l'équilibre est d'au moins 30%.

4. Système de diffusion selon la revendication 1, où le milieu aqueux est un mélange eau-solvant choisi dans le groupe comprenant les mélanges eau-alcool, eau-glycol, eau-cétone, et eau-aldéhyde.

5. Système de diffusion selon la revendication 2, où ladite membrane hydrophile est un alcool polyvinylique insoluble dans l'eau froide.

6. Système de diffusion selon la revendication 1, où ledit réservoir est un réservoir rigide muni d'un dispositif de rentrée d'air.

7. Système de diffusion selon la revendication 1, où ledit réservoir est un réservoir flexible.

8. Système de diffusion selon la revendication 1, où ladite membrane hydrophile est supportée par un substrat macroporeux.

9. Système de diffusion selon la revendication 1, où ledit agent de traitement de l'air est présent dans une émulsion huile-eau.

10. Procédé pour la libération contrôlée, uniforme, pratiquement constante d'agents volatils de traitement de l'air dans l'atmosphère consistant à mettre un système aqueux contenant ledit agent volatil de traitement de l'air dans un réservoir fermé ayant au moins une partie de sa surface de paroi constituée par une membrane hydrophile mécaniquement résistante à l'eau jusqu'à 60°C avec une teneur en eau à l'équilibre d'au moins environ 15% en poids par rapport au poids total de la membrane, ledit système aqueux étant en contact avec ladite membrane; à gonfler ladite membrane; et à permettre audit agent de traitement de l'air de diffuser à travers ladite membrane et d'être libéré dans l'atmosphère; ladite phase aqueuse passant aussi à travers ladite membrane de sorte que ledit réservoir est à peu près vide à la fin de la période de libération.

11. Procédé selon la revendication 10, caractérisé par le fait que ladite membrane hydrophile est choisie dans le groupe comprenant l'alcool polyvinylique complètement hydrolysé de poids moléculaire élevé; l'alcool polyvinylique réticulé; les hydrogels; le chlorure de polyvinyle contenant des groupes hydrophiles et ayant une charge absorbante inerte; le triacétate de cellulose; le copolyéther polycarbonate; le copolymère bloc de polyéthylène glycol-téréphtalate de polyéthylène; et les polyuréthanes hydrophiles.

12. Procédé selon la revendication 10, caractérisé par le fait que ledit milieu aqueux est un mélange eau-solvant choisi dans le groupe comprenant les mélanges eau-alcool, eau-glycol, eau-cétone et eau-aldéhyde.

13. Procédé selon la revendication 11, caractérisé par le fait que ladite membrane hydrophile est un alcool polyvinylique insoluble dans l'eau froide.

Fig.1

Fig.2

Fig.3

**O OO3 OO3**

MEMBRANE CELL AIR FLOW RATE >12 1/MIN
○ LOW
□ MEDIUM
+ HIGH

% RELEASE

TIME [ HOURS ]

Fig. 4

OPEN CUP AIR FLOW RATE <5 1/MIN
○ LOW
□ MEDIUM
+ HIGH

% RELEASE

TIME [ HOURS ]

Fig. 5

2

Fig.6

MEMBRANE CELL AIR FLOW RATE >12 1/MIN
□ TOTAL RELEASE
○ ESSENTIAL OIL RELEASE

Fig.7

OPEN CUP AIR FLOW RATE < 5 1/MIN
○ TOTAL RELEASE
□ ESSENTIAL OIL RELEASE

Fig. 8

Fig. 9

Fig. 10

Fig. 11

Fig. 12

Fig. 13